# EUROPEAN PATENT SPECIFICATION

(11) **EP 0 435 726 B1**
(45) Date of publication and mention of the grant of the patent: **09.11.1994**
(21) Application number: 90403547.4
(22) Date of filing: 12.12.1990
(51) Int. Cl.: A61J 3/07, A61J 3/10

(54) **Capsule and caplet combination**
Kombination einer Kapsel und Kaplet
Combinaison d'une capsule et une caplet

(30) Priority: 29.12.1989 US 459032
(43) Date of publication of application: 03.07.1991
(73) Proprietor: Bristol-Myers Squibb Company, New York, N.Y. 10154 (US)
(72) Inventor: Patell, Mahesh, Edison N.J.08820 (US); Frunzi, Gerard 182-26 Avon Road, N.Y. 11432 (US); Merkle, F. Henry 2217 Shawnee Path, New Jersey 07090 (US); Tencza, Thomas 31, Wagner Avenue, New Jersey 07055 (US)
(74) Representative: Hirsch, Marc-Roger

(56) References cited:
- GB-A- 1 204 580
- US-A- 2 155 444
- US-A- 2 410 110
- US-A- 3 702 653
- US-A- 4 578 075
- US-A- 4 591 500
- US-A- 4 820 524
- US-A- 4 851 230
- US-A- 4 928 840

## Description

The present invention relates to medicaments and more particularly to tamper-resistant capsules for the oral administration of therapeutic agents and to methods for the production of such capsules.

At the present time many pharmaceutical compositions are dispensed in the form of capsules. Such capsules are generally elongated, cylindrical and are often made of gelatin, starch, methyl cellulose, sugar-gelatin or gelatin-glycerin. The capsules are made in two separate parts (semicapsules or cap and body) so that they may be filled with the pharmaceutical composition, generally a powder or liquid, the two parts brought together and closed.

Capsules are preferred, by many, to other forms of medical dosage units, such as tablets, caplets (elongated tablets) or liquids. Capsules are popular because they do not have any taste and are easily swallowed. In comparison, a liquid may have a bitter or unpleasant taste and some persons have difficulty in swallowing tablets or caplets. In addition, capsules can be made in a variety of colors and sizes, are manufactured using automatic machines, and are filled by automatic capsule filling machines.

United States Patent 4,820,524 entitled "Gelatin Coated Caplets and Process for Making Same" discusses the widespread consumer preference for capsules. It also states that such powder filled capsules are not, by themselves, resistant to tampering and that the medicine in the capsule may be maliciously replaced by harmful look-alike substances. That patent suggests that a caplet may be made to look like and feel like a capsule by coating it with a gelatinous coating.

United States Patent 4,591,500 entitled "Tablet Having the Shape of a Capsule, Process and Device for Its Preparation" describes a caplet having the shape of a gelatin capsule. The caplet incorporates granules or microgranules of an active substance and is made by compression of the composition on the tablet heads, i.e., in the longitudinal direction.

United States Patent 4,851,230 entitled "Capsule Shaped Tablets" shows a pharmaceutical tablet (caplet) which is compressed into the shape of a capsule, including two heads (ogives) and a step at the junction of the two semicapsules.

The above-mentioned patents are incorporated by reference herein.

United States Patent 3,702,653 entitled "Package Means" shows a unit dose packaging, comprised of a capsule and a pharmaceutical within the capsule, independently moveable therein, and formed e.g. of a capsule.

United States Patent 2,155,444 entitled "Hexylresorcinol Capsule" shows a hexylresorcinol capsule comprised of a core of hexylresorcinol, coated with an inert material, and enclosed in a gelatin capsule, the inert material acting as a barrier between the hexylresorcinol and the gelatin.

Generally caplets are formed by compressing the composition in the width direction (horizontally) which leaves a protruding band about the caplet. It has also been suggested that caplets may be compressed in the length direction (vertically) which leaves two ledge like portions on the ends.

It would be possible to simply insert a conventional caplet in a capsule. That combined caplet-capsule is not tamper-resistant because the capsule may easily be opened and the caplet replaced with one that looks like it but is harmful.

### Summary of the Invention

In accordance with the present invention there are provided capsule-caplet combinations, in which the outer wall of a caplet inside a capsule is bound to the inner wall of the capsule, which are relatively tamper-resistant and yet may be produced at relatively low cost. There are also provided methods of producing such caplet-capsule combinations using conventional automatic tableting machinery and capsule filling machinery. The capsule-caplet is easily swallowed, like other capsules, and may contain an enteric material permitting the medicine to pass through the stomach and act in the intestine.

According to one embodiment, the present invention provides a tamper-resistant medical dosage form comprising:
a compressed caplet containing at least one medicinally active ingredient, the caplet being an elongated member having a cylindrical body portion and an exterior wall;
an elongated readily swallowable capsule comprising two semicapsules, respectively capsule cap and capsule body, each having a tubular internal wall, said capsule containing said caplet; and
characterized in that said caplet, which substantially fills the capsule, is bonded via its exterior wall to substantially the internal walls of both semicapsules to thereby provide a tamper-resistant capsule containing the caplet.

According to another embodiment, the present invention provides a tamper-resistant medical dosage oral dosage form comprising:
a caplet containing at least one medicinally active ingredient;
an edible, water vapor permeable, nontoxic layer substantially completely enveloping the caplet;
characterized in that it further comprises a hygroscopic bonding means disposed between the caplet and the layer to absorb moisture from or through the layer and to bond and conform substantially the nontoxic layer to the surface of the caplet, whereby the caplet cannot be removed from the dosage form without damaging the layer and the caplet surface.

According to yet another embodiment, the present invetion also provides a method of forming a medical dosage unit comprising:
a compressed caplet containing at least one medicinally active ingredient, the caplet being an elongated member having a cylindrical body portion and an exterior wall;
an elongated readily swallowable capsule comprising two semicapsules, respectively capsule cap and capsule body, each having a tubular internal wall, said capsule containing said caplet;
characterized in that said caplet, which substantially fills the capsule, is bonded via its exterior wall to substantially the internal walls of both semicapsules to thereby provide a tamper-resistant capsule containing the caplet;
said method comprising the steps of:
(a) preparing a compressed caplet containing at least one medicinally active ingredient;
(b) inserting the compressed caplet in the first part of a two-part preformed capsule, closing the second part of the capsule to thereby enclose the caplet, which substantially fills the capsule, within the capsule, and
(c) bonding the outer wall of the caplet to the inner walls of both parts of the capsule to form a tamper-resistant dosage form.

According to still another embodiment, the present invention provides a method of forming a tamper-resistant oral dosage unit comprising:
a caplet containing at least one medicinally active ingredient;
an edible, water vapor permeable, nontoxic layer substantially completely enveloping the caplet;
characterized in that it further comprises a hygroscopic bonding means disposed between the caplet and the layer to absorb moisture from or through the layer and to bond and conform substantially the nontoxic layer to the surface of the caplet, whereby the caplet cannot be removed from the dosage form without damaging the layer and the caplet surface;
said method comprising the steps of:
(a) preparing a caplet containing at least one medicinally active ingredient;
(b) forming a layer of hygroscopic material on the external surface of the caplet;
(c) enveloping the caplet with the said non-toxic layer; and
(d) absorbing moisture through said layer so that the hygroscopic material forms a bond between the caplet and the internal wall of said layer.

The active medical ingredient is mixed with materials to form a compressible mass. In one embodiment, the powder mass is compressed on its ends to form a caplet without a band. The caplet is coated with hygroscopic water soluble material, having adhesive properties and is inserted into one half of a pre-formed capsule (body), e.g., a water vapor permeable semicapsule. The caplet and capsule sizes are chosen so that there is contact between the external wall of the caplet and the internal wall of the capsule. The other half of the capsule is then placed on the first half, closing the capsule. In one embodiment of the invention, the capsule, with its internal caplet, is placed in a high humidity atmosphere causing the hygroscopic coating of the caplet to absorb moisture and adhere to the internal wall of the capsule thereby forming a bond between the external wall of the caplet and the internal surface of the capsule. The finished capsule-caplet is a unitary product which may not be opened without breaking the capsule shell or the caplet, thereby revealing tampering.

### Brief Description of the Drawings

The following detailed description of the invention should be taken in conjunction with the accompanying drawings. In the drawings:
Figure 1 is a side plan view of a prior art caplet showing its external band;
Figure 2A is a side plan view of the caplet used in one embodiment of the present invention;
Figure 2B is a top plan view of the caplet of Figure 2A;
Figure 3 is a side cross-sectional view of the machine used to compress the caplet shown in Figures 2A and 2B;
Figure 4 is an exploded perspective view of a capsule;
Figure 5 is a side plan view of the caplet of Figure 2A within a semicapsule; and
Figure 6 is a side plan view of the complete caplet-capsule of one embodiment of the present invention.

### Detailed Description of the Invention

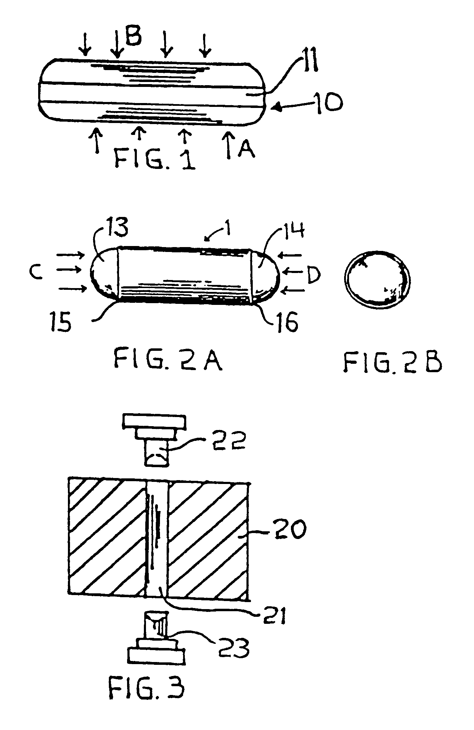

As shown in Figure 1, the conventional caplet 10 of the prior art is an elongated solid member formed from a pharmaceutical composition. It may be formed in a conventional automatic tableting machine. The caplet is generally formed by such machines by applying pressure on the sides, as shown by arrows A and B. This results in the formation of a protruding band 11, which is a ring-like protrusion about the longitudinal sides of the caplet.

The caplet 12 utilized in one embodiment of the present invention is shown in Figures 2A and 2B.

The caplet 12 is formed on automatic tableting machines, such as shown in Figure 3. The pressure is applied vertically on the top and bottom cap portions 13, 14 as shown by arrows C and D. The caplet 12 does not have a protruding band on its external wall as in the caplet shown in Figure 1. The caplet 12, due to the method of formation, has two annular flat ledge-like ring portions 15, 16 formed on its opposite cap portions 13, 14 respectively. As shown in Figure 3, the die 20 has a cylindrical bore 21 into which the compressor members 22, 23 are inserted to compress the powder mass into a caplet. These vertically compressed caplets could be designed to fit snugly into different size hard shell gelatin capsules which are available in various sizes of #000 (largest) to 00, 0, 1, 2, 3 & 4 (smallest) regular or elongated sizes.

The vertically compressed caplets are preferably formed so that their outer diameter closely approximates the internal dimensions of the capsules. This prevents the capsule shell from distorting during the bonding and packing processes and eliminates any voids and looseness of fit.

The size of the caplets, both as to their outer diameter and length, is selected to match the size of the capsules into which they are to be inserted.

The flat ring portions 15, 16 do not detract from the functioning of the caplet-capsule and do not present a problem as to consumer acceptance, since the caplet 12 is within a capsule.

The caplets of the present invention are either formed with a hygroscopic or water soluble powder, as one of their bonding materials, or formed into caplets and then coated with a hygroscopic or water soluble bonding layer.

Many hygroscopic materials are available which will absorb water and swell to fill the void volume between the outer wall of the caplet and the inner wall of the capsule and form a bond therebetween. The same materials can be employed as components in the caplet itself so that the entire caplet will expand into the void volume and bond the caplet into the capsule. The most common materials of this nature are cellulose derivatives although high molecular weight proteins and synthetic polymers are also employed. Typically useful materials which can be utilized in the practice of this invention include methyl cellulose, hydroxypropyl methylcellulose available as "Methocel" (TM of Dow Chemical, Midland, Michigan); hydroxypropyl cellulose, available as "Klucel" (TM) from Aqualon Co; Povidone; sodium carboxymethyl cellulose; carboxymethyl cellulose; "Carbopol" (TM) water soluble gel forming polyacrylic resin available from B.F. Goodrich Chemical Co. and sorbitol.

Preferably the hygroscopic deliquescent bonding material is formed as an exterior coating on the caplet. If desired, the hygroscopic material may also be an enteric, or it may be mixed with an enteric material, or the hygroscopic and enteric materials may be formed in separate layers on the caplet. The enteric coating is used to prevent the caplet from breaking up in the stomach so that the caplet passes through the stomach to the intestine, where it dissolves. A number of known enteric materials may be employed in the invention such as, cellulose acetate phthalate, hydroxypropyl methylcellulose phthalate and "Aquateric" or pseudo ethyl cellulose. A preferred enteric material is Eudragit L30D (TM) which is a copolymer that is anionic in character and based on polymethacrylic acid and acrylic acid esters. This is described by the formula. 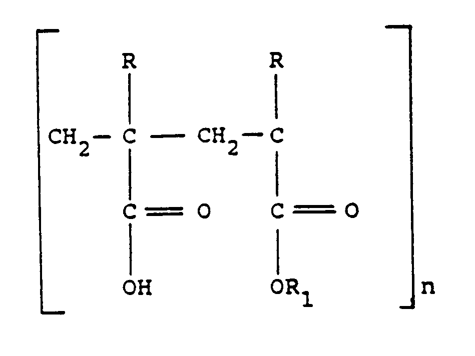
wherein:
n is a number
R is H or CH₃ and
R₁ is CH₃ or C₂H₅
the ratio of free carboxyl group to ester groups is 1:1 and the mean molecular weight of the polymer is about 250,000.
It is available from Rohm Pharma-Gmbh. Eudragit L30D (TM) is also hygroscopic.

The hygroscopic material, and the enteric material if employed, may be coated on the caplet using conventional coating methods. For example, the hygroscopic and enteric coatings may be deposited from solutions, suspensions or emulsions by dipping caplets in such liquid materials or deposited by spraying the liquids onto the caplets. If the hygroscopic and enteric materials are powders they may be dusted on the caplets and thereafter moistened, if desired.

Preferably, if the caplet is to be coated with an enteric material, it is first coated with a non-reactive film layer, for example methyl cellulose, to prevent a chemical reaction between the enteric coating and the materials of the caplet.

In one embodiment, the caplet is coated with a dry hygroscopic bonding material for example by coating or dusting or by other methods well known to the industry. It is then sprayed with a solvent, suitably alcohol/water, during the process of insertion into a gelatin semicapsule (one-half of a capsule) by an automatic capsule filling machine. The other semicapsule is then placed on the assembled caplet-semicapsule, by the automatic machine, to completely enclose the caplet in the capsule. The alcohol-water solution will dry as its vapors permeate the capsule and will form a bond between the caplet and the semicapsules.

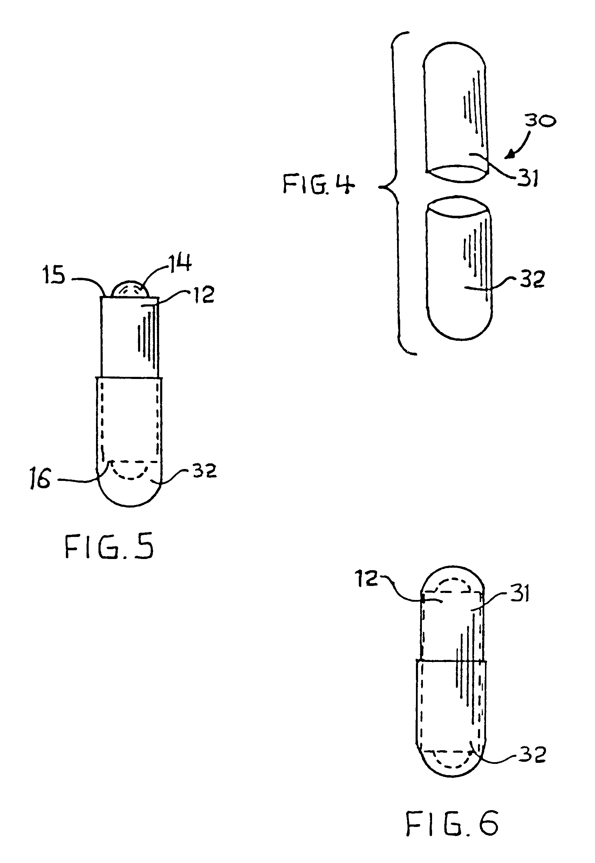

The capsule 30 shown in Figure 4, consists of two semicapsules 31, 32 each having a right-cylindrical tubular body portion and may be of conventional capsule materials. A suitable material for a capsule is gelatin although other suitable capsules may be made of other materials including gelatin-glycerin, starch, gelatin-sugar, and methylcellulose.

The material selected for the formation of the capsule may serve as the source of water for reaction with the hygroscopic material, or may be water permeable. As is known, many high molecular weight proteins such as gelatins or carbohydrates such as starches, used to form capsules, contain either absorbed water or water of hydration. Many of them can be formed into water vapor permeable capsules by procedures well known in the art.

The capsules may be of any selected color or combinations of color. For example, one semicapsule may be blue and its joined semicapsule may be clear.

The solution of hygroscopic material must be compatible with the caplet and may be sprayed or otherwise applied to the caplet. A preferred bonding solvent is a mixture of water and alcohol in the following ranges of mixtures; alcohol: water from 70:30 to 95: 5 and more preferably from 80:20 to 90:10. Alternatively, the hygroscopic bonding material may be dissolved or mixed into the solvent or other liquid carrier and thereafter coated onto the caplet in a single step.

After the assembled caplet-capsule exits the capsule filling machine, it is treated to bond the caplet to the capsule. Such bonding is preferably accomplished by the exposure to a humid atmosphere, preferably with concurrent heating. The heat/humidity conditions selected will depend principally on the properties of the bonding materials used and of the material of which the capsule is formed, e.g. the rate of throughput of water if it is water vapor permeable, or the rate at which it releases absorbed water or water of hydration. For most materials employed in the preparation of sealed capsules, typical conditions range from about 25°C to about 75°C at a specific humidity of from about 30% to about 100% during a period of from about 2 to about 24 hours. The preferred ranges are about 40°C to about 45°C at a specific humidity of about 75% to about 85% during a period of about 8 to about 12 hours.

In an alternative embodiment, illustrated in Example I below, after the assembled caplet-capsule exits the capsule filling machine, a band of water-alcohol solution is applied to a ring at the free edge of the outer semicapsule (capsule cap). The solution, by capilliary action, flows to the juncture of the caplet exterior wall and capsule interior wall, wets the hygroscopic material and bonds the caplet to the interior walls of both semicapsules. In addition, the solution spreads by capillary action to the overlapped portions of the two semicapsules and, when dried, bonds these overlapped portions. Such bonding together of the two semicapsules helps make the caplet-capsule tamper resistent. The band of water-alcohol solution may be applied by modifying commercially available automatic capsule banding equipment.

### EXAMPLE I

A combination of analgesic ingredients consisting of 500 mg. of acetaminophen and 65 mg. of caffeine was compressed into a right-sided cylinder with rounded ends using special capsule shape tooling. The caplet dimensions were: length 0.826" and diameter 0.262". This tablet shape was selected to provide an exact fit to match the inner dimensions of a #0 hard gelatin capsule shell. The caplets (core caplets) were then coated with a film coating of hydroxypropyl methylcellulose and inserted into the capsule body by hand. The closed capsule was then bonded using an Elanco laboratory model "Quali-Seal (TM) hard gelatin capsule banding machine using an 80:20 ethyl alcohol water solution. The solution is placed, by the banding machine, at the junction of the cap and body. The solution flows, by capillary action, between the caplet and cap and body and also between the overlapped portions of the cap and body.

An alternative to the aqueous/alcoholic solution is the use, in the same manner, of plain water containing a small amount of surfactant (1/4 to 1% Tween 20); or bonding agents such as Povidone, Methocel or Glycerin (1-5%). The solution was applied by the machine at the juncture of the two semicapsules i.e., at the juncture of the cap and body and flows, by capillary action, in the same manner as the above-described alcohol-water solution.

Immediately subsequent to the application of the bonding solution, the capsules are exposed for a brief period to the drying effects of a microwave oven. A Litton-Generation II oven (approximately 1 cubic foot) at a high power setting for a period of 40 seconds was used. This treatment in a microwave oven bonds the semicapsules (cap and body) and the core caplet to the inner surfaces of the capsule shell and shrinks the capsule shell making it impossible to separate and remove the core caplet without destroying the dosage unit.

The resulting bonded and sealed gelatin capsules prepared in this Example were judged to be pharmaceutically elegant with the physical attributes and appearance of a hard gelatin capsule and with the additional benefit of a solid core caplet having a bonded gelatin coating.

The drying, described above, occurs in a microwave oven. Alternatively, in commercial production, the drying may be accomplished by other drying processes, such as fluid bed drying or oven drying.

In an alternative embodiment, illustrated in Example II below, the moisture content of the semicapsules is increased resulting in an expansion of the inner diameters of the semicapsules. The moisture content may be increased by placing the caplet filled capsules in an environment of from about 25°C to about 75°C and a relative humidity of from about 30% to about 100% during a period of about 2 hours to about 24 hours. The preferred ranges are about 30°C to about 45°C and a relative humidity of about 75% to about 90% during a period of about 6 hours to about 12 hours. For example, the moisture content of gelatin semicapsules may be increased from its equilibrium moisture 14%-16% to about 18%. The caplets are inserted into the untreated semicapsules, the semicapsules are closed and the caplet-capsules are raised in their moisture content resulting in a shrinkage of the capsule around the caplet, and then dried to their equilibrium moisture, so that the capsules tightly enclose and adhere to their caplets.

### EXAMPLE II

A combination of analgesic ingredients consisting of 500 mg. of acetaminophen and 65 mg of caffeine was compressed into a cylinder with rounded ends using special shaped tooling. The caplet dimensions were: length 0.826 "and diameter 0.262". This caplet shape was designed and selected to provide an exact fit to match the inner dimensions of a #0 hard gelatin capsule shell.

Both uncoated and film coated core caplets were inserted into empty hard gelatin capsule shells by hand and the cap and body shells were closed to form closed capsules. The film coating consisted of hydroxypropyl methylcellulose and other minor film coating additives. The closed capsules were then subjected to an environmental condition of 90% RH at 30°C in a temperature/humidity chamber for a period of 12 hours. This treatment allowed the caplet filled gelatin shells to increase their moisture to a higher moisture content of approximately 18%.

The treated capsules were removed from the temperature/humidity chamber and allowed to reequilibrate on a laboratory bench top to ambient temperature and humidity conditions. This process of reequilibration or drying caused the capsule shells to contract and shrink to the contours of the core caplet and resulted in a permanent bond between the inner surfaces of the capsule shell and the core caplet as well as bonding the overlapped cap and body portions of the capsule shell.

Caplets having a longitudinal protruding band may be also used in the present invention. For example the space between the non-band portion of the external wall of the caplet and the internal wall of the capsule may be filled with the water expanded hygroscopic material.

While this invention has been described principally as applied to caplets such as caplet 12, it will be apparent to those skilled in the art that it is equally applicable to caplets such as caplet 10 which has a longitudinal protruding band.

If desired a band of gelatin, or other swallowable material, may be fastened to the caplet filled capusle at the cap/body juncture to provide additional tamper resistance. In the event of tampering, the removal of the band from a capsule would be easily recognized by the consumer.

In addition, the capsules/may be made transparent so that information, such as names, codes, and logos, printed or embossed on the caplets, may be read by consumers.

## Claims

1. A tamper-resistant medical dosage form comprising:
a compressed caplet (12) containing at least one medicinally active ingredient, the caplet being an elongated member having a cylindrical body portion and an exterior wall;
an elongated readily swallowable capsule (30) comprising two semicapsules (31,32), respectively capsule cap and capsule body, each having a tubular internal wall, said capsule containing said caplet (12); and
characterized in that said caplet (12), which substantially fills the capsule, is bonded via its exterior wall to substantially the internal walls of both semicapsules (31, 32) to thereby provide a tamper-resistant capsule (30) containing the caplet (12).

2. A tamper-resistant medical dosage oral dosage form comprising:
a caplet (12) containing at least one medicinally active ingredient;
an edible, water vapor permeable, nontoxic layer substantially completely enveloping the caplet (12);
characterized in that it further comprises a hygroscopic bonding means disposed between the caplet and the layer to absorb moisture from or through the layer and to bond and conform substantially the nontoxic layer to the surface of the caplet, whereby the caplet cannot be removed from the dosage form without damaging the layer and the caplet surface.

3. The dosage form as in claim 1 and comprising a hygroscopic material to bond said caplet exterior wall to said capsule internal walls.

4. The dosage form as in claims 2 or 3 wherein the hygroscopic material is incorporated in the caplet as a binding agent.

5. The dosage form as in claims 1 or 2 wherein said caplet is coated with a layer of enteric material.

6. A method of forming a medical dosage unit according to claim 1, in which a caplet (12) containing at least one medicinally active ingredient is enclosed in an edible capsule (30), comprising the steps of:
(a) preparing a compressed caplet containing at least one medicinally active ingredient;
(b) inserting the compressed caplet in the first part of a two-part preformed capsule (31, 32), closing the second part of the capsule to thereby enclose the caplet, which substantially fills the capsule, within the capsule, and
(c) bonding the outer wall of the caplet to the inner walls of both parts of the capsule to form a tamper-resistant dosage form.

7. The method of claim 6, wherein step (a) consists in forming an elongated caplet (12) having an imaginary axis, a right-cylindrical body portion and two opposite ends (13, 14), by placing a powder mass containing the medicinally active ingredient in a confined die (20) whose internal wall is cylindrical and forms the caplet body portion, and compressing the powder mass along the caplet axis on the caplet ends to form the compressed caplet;

8. The method of claim 6 or 7, further including the step of adding a hygroscopic material to the powder mass prior to compression.

9. The method of claim 6 or 7, further including the step of coating a hygroscopic material on the compressed caplet.

10. A method of forming a tamper-resistant oral dosage unit according to claim 2 in which a caplet containing at least one medicinally active ingredient is enclosed in an edible, water vapor permeable, and non-toxic layer, comprising the steps of:
(a) preparing a caplet containing at least one medicinally active ingredient;
(b) forming a layer of hygroscopic material on the external surface of the caplet;
(c) enveloping the caplet with the said non-toxic layer; and
(d) absorbing moisture through said layer so that the hygroscopic material forms a bond between the caplet and the internal wall of said layer.

11. The method of claims 6 or 10, and including the step of coating an enteric material on the surface of the caplet after it is compressed.

## Patentansprüche

1. Mißbrauchsichere, medizinische Darreichungsform, umfassend:
ein komprimiertes Kaplet (12), welches mindestens einen medikamentös wirksamen Bestandteil enthält, welches Kaplet ein längliches Teil mit einem zylindrischen Gehäuseabschnitt und einer Außenwandung ist;
eine ländliche, leicht schluckbare Kapsel (30), umfassend zwei Halbkapseln (31, 32), bzw. Kapselkappe und Kapselgehäuse, die jede eine röhrförmige Innenwandung haben, welche Kapsel das Kaplet (12) enthält; und
dadurch gekennzeichnet, daß das Kaplet (12), welches die Kapsel im wesentlichen ausfüllt, über seine Außenwandung mit den Innenwandungen beider Halbkapseln (31, 32) im wesentlichen verbunden ist, um dadurch eine mißbrauchsichere, das Kaplet (12) enthaltende Kapsel (30) zu schaffen.

2. Mißbrauchsichere, medizinische, orale Darreichungsform, umfassend:
ein Kaplet (12), das mindestens einen medikamentös wirksamen Bestandteil enthält;
eine genießbare, wasserdampfdurchlässige, nichttoxische Schicht, die das Kaplet (12) im wesentlichen vollständig umhüllt;
dadurch gekennzeichnet, daß sie ferner ein hygroskopisches Bindemittel aufweist, welches zwischen dem Kaplet und der Schicht angeordnet ist, um Feuchtigkeit von der oder durch die Schicht zu absorbieren und um die nichttoxische Schicht an der Oberfläche des Kaplet im wesentlichen zu binden und anzupassen, wodurch das Kaplet aus der Darreichungsform nicht entfernt werden kann, ohne die Schicht und die Kapletoberfläche zu beschädigen.

3. Darreichungsform nach Anspruch 1 und umfassend ein hygroskopisches Material, um die Außenwandung des Kaplets mit den Innenwandungen der Kapsel zu verbinden.

4. Darreichungsform nach Anspruch 2 oder 3, bei welcher das hygroskopische Material in dem Kaplet als ein Bindemittel beigemengt ist.

5. Darreichungsform nach Anspruch 1 oder 2, bei welcher das Kaplet mit einer Schicht aus dünndarmlöslichem Material beschichtet ist.

6. Verfahren zum Bilden einer medizinischen Darreichungseinheit nach Anspruch 1, bei welchem ein Kaplet (12), welches mindestens einen medikamentös wirksamen Bestandteil enthält, in einer genießbaren Kapsel (30) eingeschlossen ist, umfassend die Schritte:
(a) Zubereiten eines komprimierten Kaplets, welches mindestens einen medikamentös wirksamen Bestandteil enthält;
(b) Einsetzen des komprimierten Kaplets in den ersten Teil einer zweiteiligen, vorgeformten Kapsel (31, 32), Schließen des zweiten Teils der Kapsel, um dadurch das Kaplet, das die Kapsel im wesentlichen ausfüllt, im Inneren der Kapsel einzuschließen; und
(c) Binden der Außenwandung des Kaplets an den Innenwandungen beider Teile der Kapsel, um eine mißbrauchsichere Darreichungsform zu bilden.

7. Verfahren nach Anspruch 6, bei welchem Schritt (a) im Bilden eines länglichen Kaplets (12) mit einer imaginären Achse, einem geraden, zylindrischen Gehäuseabschnitt und zwei gegenüberliegenden Enden (13,14) besteht, indem eine den medikamentös wirksamen Bestandteil enthaltende Pulvermasse in eine geschlossene Form (20) gegeben wird, deren Innenwandung zylindrisch ist und den Kaplet-Gehäuseabschnitt bildet; sowie Komprimieren der Pulvermasse entlang der Kaplet-Achse an den Kaplet-Enden, um das komprimierte Kaplet zu bilden.

8. Verfahren nach Anspruch 6 oder 7, ferner einschließend den Schritt des Zusetzens eines hygroskopischen Materials zur Pulvermasse vor der Kompression.

9. Verfahren nach Anspruch 6 oder 7, ferner einschließend den Schritt des Auftragens eines hygroskopischen Materials auf dem komprimierten Kaplet.

10. Verfahren zum Bilden einer mißbrauchsicheren, oralen Darreichungseinheit nach Anspruch 2, bei welchem ein den medikamentös wirksamen Bestandteil enthaltendes Kaplet eingeschlossen ist in einer genießbaren, wasserdampfdurchlässigen und nichttoxischen Schicht, umfassend die Schritte:
(a) Zubereiten eines Kaplets, welches mindestens einen medikamentös wirksamen Bestandteil enthält;
(b) Bilden einer Schicht aus hygroskopischem Material auf der Außenfläche des Kaplets;
(c) Umhüllen des Kaplets mit der genannten nichttoxischen Schicht und
(d) Absorbieren von Feuchtigkeit durch die Schicht, so daß das hygroskopische Material eine Bindung zwischen dem Kaplet und der Innenwandung der Schicht bildet.

11. Verfahren nach Anspruch 6 oder 10 und einschließend den Schritt des Auftragens eines dünndarmlöslichen Materials auf die Oberfläche des Kaplets, nachdem es komprimiert wurde.

## Revendications

1. Une forme de dosage médical résistant à toute modification comprenant:
- un cachet compressé (12) contenant au moins un ingrédient actif du point de vue médicinal, le cachet étant un élément allongé comportant une partie de corps cylindrique et une paroi extérieure;
- une gélule (30) allongée susceptible d'être facilement avalée comprenant deux demi-gélules (31, 32), respectivement un capuchon de gélule et un corps de gélule, chacun comportant une paroi tubulaire interne, ladite gélule contenant ledit cachet (12);
caractérisée en ce que ledit cachet (12) qui remplit substantiellement la gélule, est lié substantiellement via sa paroi externe aux parois internes des deux demi-gélules (31, 32) de façon à former une gélule (30) contenant le cachet (12) et résistant à toute modification.

2. Une forme de dosage médical oral résistante à toute modification comprenant:
- un cachet (12) contenant au moins un ingrédient actif du point de vue médicinal;
- une couche non toxique perméable à la vapeur d'eau, comestible, enveloppant pratiquement la totalité du cachet;
caractérisée en ce qu'elle comprend de plus des moyens de liaison microscopiques disposés entre le cachet et la couche pour absorber l'humidité de la couche et lier et adapter la couche non toxique sensiblement à la surface du cachet, de façon à ce que le cachet (12) ne puisse pas être retiré de la forme de dosage sans endommager la couche et la surface du cachet (12).

3. La forme de dosage selon la revendication 1, comprenant un matériau hygroscopique pour lier ladite paroi externe du cachet aux parois internes de ladite gélule.

4. La forme de dosage selon les revendications 2 ou 3, dans laquelle le matériau hygroscopique est incorporé dans le cachet en tant qu'élément de liaison.

5. La forme de dosage selon les revendications 1 ou 2, dans laquelle ledit cachet est revêtu d'une matière entérique.

6. Un procédé de formation d'une dose médicale unitaire selon la revendication 1, dans lequel un cachet (12) contenant au moins un ingrédient actif du point de vue médicinal est enfermé dans une gélule comestible (30) comprenant les étapes de:
a) préparation d'un cachet comprimé contenant au moins un ingrédient actif du point de vue médicinal;
b) l'insertion du cachet comprimé dans la première partie des deux parties de la gélule préformée, la fermeture de la seconde partie de la gélule de façon à enclore dans la gélule le cachet, qui remplit sensiblement la gélule; et
c) liaison de la paroi externe du cachet aux parois internes des deux parties de la gélule pour former une forme de dosage résistant à toute modification.

7. Le procédé selon la revendication 6, dans lequel l'étape (a) consiste en la formation d'un cachet allongé (12) présentant un axe imaginaire, une partie de corps cylindrique droite et deux extrémités opposées (13, 14), à plaçer une masse de poudre contenant l'ingrédient actif du point de vue médical dans un moule confiné (20) dont la paroi interne est cylindrique et forme la partie du corps du cachet, et à compresser la masse de poudre, selon l'axe du cachet, par les extrémités du cachet pour former un cachet compressé.

8. Le procédé selon la revendication 6 ou 7, incluant de plus l'étape d'addition d'une matière hygroscopique à la masse de poudre avant la compression.

9. Le procédé selon la revendication 6 ou 7, incluant de plus l'étape de revêtement d'un matériau hygroscopique sur le cachet comprimé.

10. Un procédé de formation d'un dosage unitaire oral résistant à toute modification selon la revendication 2, dans lequel un cachet contenant au moins un ingrédient actif du point de vue médicinal est renfermé dans une couche non toxique, perméable à la vapeur d'eau et comestible, comprenant les étapes de:
a) préparation d'un cachet contenant au moins un ingrédient actif du point de vue médicinal;
b) formation d'une couche d'une matière hygroscopique sur la surface externe du cachet;
c) revêtement du cachet par ladite couche non toxique; et
d) absorption de l'humidité à travers ladite couche de façon à ce que la matière hygroscopique forme une liaison entre le cachet et la paroi interne de ladite couche.

11. Le procédé selon les revendications 6 ou 10, incluant l'étape de revêtement d'une matière entérique sur la surface du cachet après sa compression.
